(19)

**Europäisches Patentamt**

**European Patent Office**

**Office européen des brevets**

(11) **EP 3 786 976 A1**

(12) **EUROPEAN PATENT APPLICATION**

(43) Date of publication:
**03.03.2021 Bulletin 2021/09**

(51) Int Cl.:
**G16H 40/67** *(2018.01)*     **G16H 50/20** *(2018.01)*

(21) Application number: **19461575.3**

(22) Date of filing: **29.08.2019**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicants:
- **Politechnika Lodzka**
  **90-924 Lodz (PL)**
- **Uniwersytet Medyczny w Lodzi**
  **90-419 Lodz (PL)**
- **Instytut Medycyny Pracy im. prof. dr med. Jerzego**
  **Nofera w Lodzi**
  **91-348 Lodz (PL)**
- **Labo Clinic Sp. z o.o.**
  **10-437 Olsztyn (PL)**
- **Platinum Seed Incubator Sp. z o.o.**
  **87-100 Torun (PL)**

(72) Inventors:
- **TYLMAN, Wojciech**
  **93-347 Lodz (PL)**
- **KOTAS, Rafal**
  **97-400 Beichatow (PL)**
- **MARCINIAK, Pawel**
  **92-524 Lodz (PL)**
- **KAMINSKI, Marek**
  **93-150 Lodz (PL)**
- **SAKOWICZ, Bartosz**
  **93-534 Lodz (PL)**
- **SZERMER, Michal**
  **93-352 Lodz (PL)**
- **JANC, Magdalena**
  **94-116 Lodz (PL)**
- **ZAMYSLOWSKA-SZMYTKE, Ewa**
  **91-502 Lodz (PL)**
- **JOZEFOWICZ-KORCZYNSKA, Magdalena**
  **93-191 Lodz (PL)**

(74) Representative: **Kancelaria Eupatent.pl Sp. z.o.o**
  **Ul. Kilinskiego 185**
  **90-348 Lodz (PL)**

(54) **DATA PROCESSING IN A SYSTEM FOR DYNAMIC POSTUROGRAPHY**

(57) A system for dynamic posturography of a person, the system comprising: a wearable device (502) comprising: a data bus (101) communicatively coupled to a memory (104); other components of the wearable device communicatively coupled to the system bus (101) so that they may be managed by a controller (105); a communication module (102) to communicate via a wireless communication channel; a 3-axis inertial sensor (103) reporting data to the controller (105); and a clock module (107) configured to provide timing for sampling data from the 3-axis inertial sensor (103) at a preferred rate; and an external device (501) configured to communicate with the wearable device (502) via the wireless communication channel. Said controller (105) of the wearable device (502) is configured to execute the following steps: receiving data (201) from a 3-axis inertial sensor (103); and filtering of the received data (202). Said external device (501) is configured to receive from the wearable device (502) the filtered received data and to execute the following steps: determining angular speed (203) of the wearable device (502); calculating (204) a projection of a center of gravity onto a base surface, on which the person is positioned, wherein $x = h \sin(\alpha)$ and $y = h \sin(\beta)$ wherein h denotes a height at which the device is mounted, while the angles $\alpha$ and $\beta$ are the roll and pitch angles of the angular position of the device calculated based on the data from the 3-axis inertial sensor; calculating (205) movement parameters comprising angular speeds and trajectory and surface areas of the wearable device (502); and comparing (206) the results of the movement parameters with assumed norms and outputting a result of the comparison as the dynamic posturography parameters.

EP 3 786 976 A1

**501** External device (PC, server)

Start
Data request
Samples data

**502** Wearable device (sensors, Fig. 1)

Fig. 5A

**Description**

**TECHNICAL FIELD**

The present invention relates to data processing in a system for dynamic posturography.

**BACKGROUND OF THE INVENTION**

Posturography is a general term that covers all the techniques used to quantify postural control in upright stance in either static or dynamic conditions. Posturography is also an assessment technique used to objectively quantify and differentiate among a variety of possible sensory, motor, and central adaptive impairments to balance control.

Posturography is therefore a technique used in evaluating and monitoring of persons with balance problems. Results of dynamic postugorraphy measurements can be used as input data to various diagnosis procedures to diagnose balance disorders.

Prior art defines a publication of US20140081177A1 entitled "Posturographic system using a balance board", which discloses a stabilometric system is provided that uses a balance platform to detect problems in the vestibular system via data capture, data visualization and mathematical analysis of data, the system having means for data capture that obtain customized records and store data resulting from the readings of the sensors of the balance platform, means for displaying the data obtained using stabilometric tests on a screen that is controlled by a computer, and means for processing the data obtained from the measurements.

It would be advantageous to provide a dynamic posturography system that would not require an expensive equipment such as the balance platform of the prior art, but a simpler device instead.

The aim of the development of the present invention is an improved and cost effective method and apparatus for dynamic posturography, including data transmission features.

**SUMMARY AND OBJECTS OF THE PRESENT INVENTION**

The object of the invention is a system for dynamic posturography of a person, the system comprising: a wearable device comprising: a data bus communicatively coupled to a memory; other components of the wearable device communicatively coupled to the system bus so that they may be managed by a controller; a communication module to communicate via a wireless communication channel; a 3-axis inertial sensor reporting data to the controller; a clock module configured to provide timing for sampling data from the 3-axis inertial sensor at a preferred rate; and an external device configured to communicate with the wearable device via the wireless communication channel. Said controller of the wearable device is configured to execute the following steps: receiving data from a 3-axis inertial sensor; and filtering of the received data. Said external device is configured to receive from the wearable device the filtered received data and to execute the following steps: determining angular speed of the wearable device; calculating a projection of a center of gravity onto a base surface, on which the person is positioned, wherein $x = h \sin(\alpha)$ and $y = h \sin(\beta)$ wherein h denotes a height at which the device is mounted, while the angles $\alpha$ and $\beta$ are the roll and pitch angles of the angular position of the device calculated based on the data from the 3-axis inertial sensor; calculating movement parameters comprising angular speeds and trajectory and surface areas of the wearable device; and comparing the results of the movement parameters with assumed norms and outputting a result of the comparison as the dynamic posturography parameters.

Another object of the invention is a method for dynamic posturography of a person by means of a wearable device mounted to a person's body, the method comprising the steps of: attaching the wearable device to the person's body. At the wearable device : receiving data from a 3-axis inertial sensor mounted in the device; and filtering of the received data. At an external device configured to communicate with the wearable device via the wireless communication channel: receiving the filtered data from the wearable device; determining angular speed of the wearable device; calculating coordinates (x, y) of a projection of a center of gravity onto a base surface, on which the person is positioned, wherein $x = h \sin(\alpha)$ and $y = h \sin(\beta)$ wherein h denotes a height at which the device is mounted, while the angles $\alpha$ and $\beta$ are the roll and pitch angles of the angular position of the device calculated based on the data from the 3-axis inertial sensor; calculating movement parameters comprising angular speeds and trajectory and surface areas of the wearable device; and comparing the results of the movement parameters with assumed norms and outputting a result of the comparison as the dynamic posturography parameters.

Preferably, said sampling rate is 20 Hz.

Preferably, the system further comprises a magnetometer.

Preferably, said filtering is effected by a low-pass filter operating in a continuous manner, which is a Butterworth, 2nd-order, digital filter having a cutoff frequency of 10 Hz.

Preferably, said step of calculating movement parameters is effected in the following manner:

- current angular speed in the XY plane

$$\frac{hypot\big(pitch(i)-pitch(i-1),roll(i)-roll(i-1)\big)}{T}$$

where hypot(x,y) is $\sqrt{x^2+y^2}$

- trajectory length $\sum_{i=1}^{N} hypot\big(x(i)-x(i-1),y(i)-y(i-1)\big)$, wherein N is a samples count

- plane area $\sum_{i=1}^{N} |\big(\big(x(i-1)-x_0\big)\big(y(i)-y_0\big)-\big(x(i)-x_0\big)\big(y(i-1)-y_0\big)\big)|$, wherein $x_0$ and $y_0$ is a geometric center of said trajectory.

Preferably, the step of determining angular speed is effected by executing the following steps: quaternion initialization; reading data from sensors (103, 107); data normalization; determining a reference direction of Earth's magnetic field; determining a correction value for the quaternion based on their current value and readings from the sensors; normalizing the correction value and modifying said quaternion using the correction value; normalizing the quaternion and determining angular position of the sensor.

Preferably, said determining a correction value comprises the following equations:

- $s_1 = -2q_3(2q_2q_4 - 2q_1q_3 - a_x) + 2q_2(2q_1q_2 + 2q_3q_4 - a_y) - 2b_zq_3(2b_x(0.5 - q_3q_3 - q_4q_4) + 2b_z(q_2q_4 - q_1q_3) - m_x) + (-2b_xq_4 + 2b_zq_2)(2b_x(q_2q_3 - q_1q_4) + 2b_z(q_1q_2 + q_3q_4) - m_y) + 2b_xq_3(2b_x(q_1q_3 + q_2q_4) + 2b_z(0.5 - q_2q_2 - q_3q_3) - m_z)$
- $s_2 = 2q_4(2q_2q_4 - 2q_1q_3 - a_x) + 2q_1(2q_1q_2 + 2q_3q_4 - a_y) - 4q_2(1 - 2q_2q_2 - 2q_3q_3 - a_z) + 2b_zq_4(2b_x(0.5 - q_3q_3 - q_4q_4) + 2b_z(q_2q_4 - q_1q_3) - m_x) + (2b_xq_3 + 2b_zq_1)(2b_x(q_2q_3 - q_1q_4) + 2b_z(q_1q_2 + q_3q_4) - m_y) + (2b_xq_4 - 4b_zq_2)(2b_x(q_1q_3 + q_2q_4) + 2b_z(0.5 - q_2q_2 - q_3q_3) - m_z)$
- $s_3 = -2q_1(2q_2q_4 - 2q_1q_3 - a_x) + 2q_4(2q_1q_2 + 2q_3q_4 - a_y) - 4q_3(1 - 2q_2q_2 - 2q_3q_3 - a_z) + (-4b_xq_3 - 2b_zq_1)(2b_x(0.5 - q_3q_3 - q_4q_4) + 2b_z(q_2q_4 - q_1q_3) - m_x) + (2b_xq_2 + 2b_zq_4)(2b_x(q_2q_3 - q_1q_4) + 2b_z(q_1q_2 + q_3q_4) - m_y) + (2b_xq_1 - 4b_zq_3)(2b_x(q_1q_3 + q_2q_4) + 2b_z(0.5 - q_2q_2 - q_3q_3) - m_z)$
- $s_4 = 2q_2(2q_2q_4 - 2q_1q_3 - a_x) + 2q_3(2q_1q_2 + 2q_3q_4 - a_y) + (-4bxq4 + 2b_zq_2)(2b_x(0.5 - q_3q_3 - q_4q_4) + 2b_z(q_2q_4 - q_1q_3) - m_x) + (-2b_xq_1 + 2b_zq_3)(2b_x(q_2q_3 - q_1q_4) + 2b_z(q_1q_2 + q_3q_4) - m_y) + 2b_xq_2(2b_x(q_1q_3 + q_2q_4) + 2b_z(0.5 - q_2q_2 - q_3q_3) - m_z)$

Preferably, said normalizing the correction value and modifying said quaternion comprises the following equations:

- $\delta q_1 = 0.5(-q_2g_x - q_3g_y - q_4g_z) - s_1f_s\beta;$
- $\delta q_2 = 0.5(q1g_x + q_3g_z - q_4g_y) - s_2f_s\beta;$
- $\delta q_3 = 0.5(q_1g_y - q_2g_z + q_4g_x) - s_3f_s\beta;$
- $\delta q_4 = 0.5(q_1g_z + q_2g_y - q_3g_x) - s_4f_s\beta;$
- wherein $f_s$ - is a sampling frequency, $\beta$ - is an attenuation coefficient (0-1).

**BRIEF DESCRIPTION OF THE DRAWINGS**

These and other objects of the invention presented herein, are accomplished by providing a system and method for data transmission in dynamic posturography. Further details and features of the present invention, its nature and various advantages will become more apparent from the following detailed description of the preferred embodiments shown in a drawing, in which:

Fig. 1 presents a diagram of a wearable device for dynamic posturography according to the present invention;
Fig. 2 presents a diagram of the method for dynamic posturography according to the present invention;
Fig. 3 presents a simplified diagram of a principle of determining coordinates of a projection of a centre of gravity onto a base plane/surface; and
Fig. 4 presents a method for determining an angular speed;
Figs. 5A-B depict elements related to data transmission scheme according to the present invention;
Fig. 6 shows a data transmission scheme;

Fig. 7 presents the positions at which the device can be mounted on the person's body.

**NOTATION AND NOMENCLATURE**

Some portions of the detailed description which follows are presented in terms of data processing procedures, steps or other symbolic representations of operations on data bits that can be performed on computer memory. Therefore, a computer executes such logical steps thus requiring physical manipulations of physical quantities.

Usually these quantities take the form of electrical or magnetic signals capable of being stored, transferred, combined, compared, and otherwise manipulated in a computer system. For reasons of common usage, these signals are referred to as bits, packets, messages, values, elements, symbols, characters, terms, numbers, or the like.

Additionally, all of these and similar terms are to be associated with the appropriate physical quantities and are merely convenient labels applied to these quantities. Terms such as "processing" or "creating" or "transferring" or "executing" or "determining" or "detecting" or "obtaining" or "selecting" or "calculating" or "generating" or the like, refer to the action and processes of a computer system that manipulates and transforms data represented as physical (electronic) quantities within the computer's registers and memories into other data similarly represented as physical quantities within the memories or registers or other such information storage.

A computer-readable (storage) medium, such as referred to herein, typically may be non-transitory and/or comprise a non-transitory device. In this context, a non-transitory storage medium may include a device that may be tangible, meaning that the device has a concrete physical form, although the device may change its physical state. Thus, for example, non-transitory refers to a device remaining tangible despite a change in state.

As utilized herein, the term "example" means serving as a non-limiting example, instance, or illustration. As utilized herein, the terms "for example" and "e.g." introduce a list of one or more non-limiting examples, instances, or illustrations.

**DESCRIPTION OF EMBODIMENTS**

The present invention is not based on a balance platform and utilizes an inertia sensor (optionally supported by a magnetic field sensor) allowing for measuring its relative positioning as well as its angular position. Said sensor(s) affixed to a person's body facilitates precise determination of positioning of a selected body part and recording of changes of said positioning in time. A set of such sensors will in turn result in an alternative method for executing dynamic posturography.

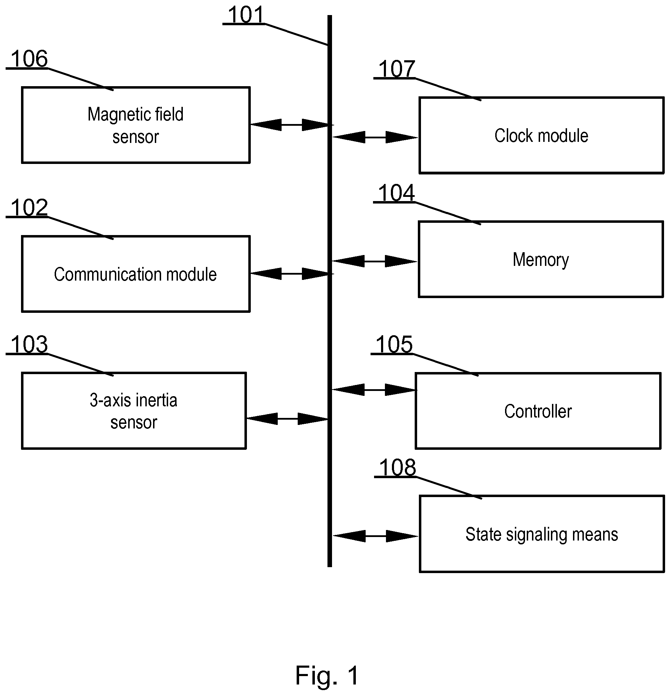

Fig. 1 presents a diagram of a wearable device for use in a system according to the present invention. The device is preferably supplied with power from a battery and may be realized using dedicated components or custom made FPGA or ASIC circuits. The wearable device comprises a data bus 101 communicatively coupled to a memory 104. Additionally, other components of the device are communicatively coupled to the system bus 101 so that they may be managed by a controller 105.

The memory 104 may store computer program or programs executed by the controller 105 in order to execute steps of the method according to the present invention.

A communication module 102 will typically be user to communicate with an external machine such as a PC, a smartphone, a tablet or the like. The communication link may be such as Wi-Fi, Bluetooth or the like.

The device is preferably sufficiently small and light in order to be mounted on person's body while not hindering any movement of the person.

A 3-axis inertial sensor 103 is implemented in the device and reports data to the controller 105. Such sensor is sometimes referred to as an inertial measurement unit (IMU) and is an electronic device that measures and reports forces, angular rate, and sometimes a level of a surrounding magnetic field (local vector of a magnetic field). The device uses a combination of accelerometers and gyroscopes, sometimes also magnetometers 106.

A clock module 107 may be used for sampling data from the sensors 105, 106 at a preferred rate, which is 20 Hz.

The controller 105 may, at least partially, process the received data from the sensors in order to limit transmission data load.

A signaling means may be present 108 that allow to signal a state of the system to a user. The signaling may be visual or audio wherein visual options may be as simple as LEDs and as sophisticated as small touch screens.

In an alternative embodiment the main sensors pair 103, 106 may be an LSM9DS1 circuit which is supplemented by a reserve LSM6DSM inertia circuit. The reserve inertia circuit is useful because in some cases when in upside - down orientation, the observed levels of noise are greatly increased. Said reserve inertia circuit may be mounted in an opposite orientation to the main sensor thereby allowing for selection of data readings having less noise.

Test embodiments of the present invention have shown that a printed circuit board comprising all the elements of the wearable device may be as small as 3 x 3 cm (excluding the power source).

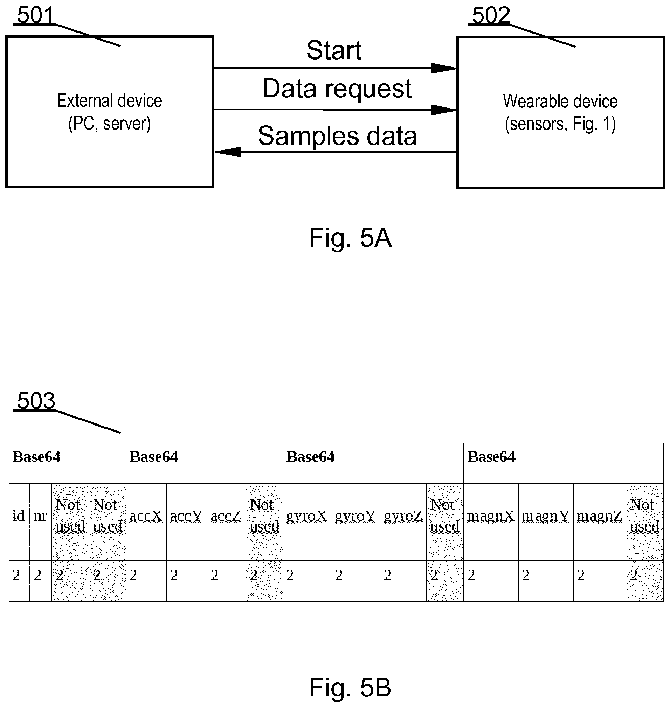

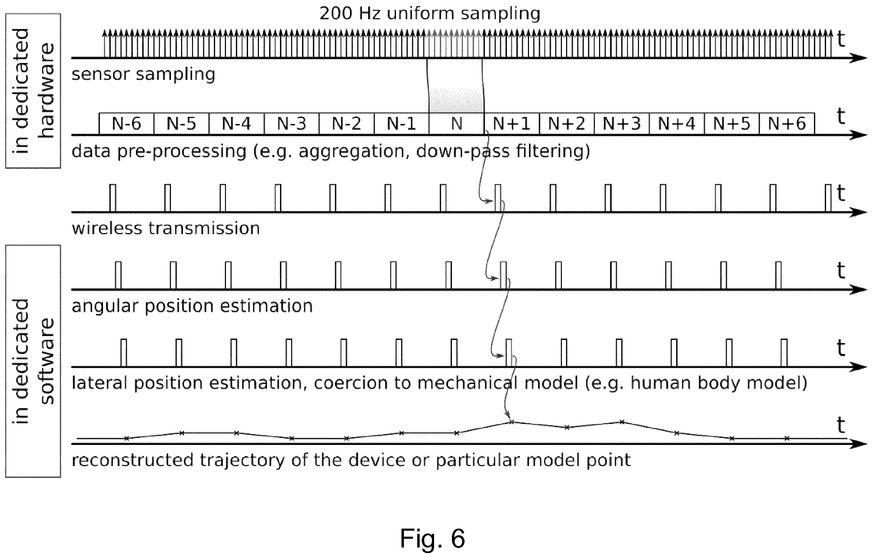

Typically, the wearable device will operate in a loop comprising general steps of (a) data acquisition, (b) data processing and (c) delivery of results (preferably to a paired external device, according to the procedure described in Fig. 5A, 5B, 6).

The transmitted data may comprise:

id - identifier of the device, preferably unique identifier;
sno - sample number;
accX - acceleration in the X axis registered by the accelerator of the sensor 103;
accY - acceleration in the Y axis registered by the accelerator of the sensor 103;
accZ - acceleration in the Z axis registered by the accelerator of the sensor 103;
gyroX - angular speed in the X axis of a gyroscope of the sensor 103;
gyroY - angular speed in the Y axis of a gyroscope of the sensor 103;
gyroZ - angular speed in the Z axis of a gyroscope of the sensor 103;
magnX - optionally registered magnetic field in the X axis of a magnetometer sensor 107;
magnY - optionally registered magnetic field in the Y axis of a magnetometer sensor 107;
magnZ - optionally registered magnetic field in the Z axis of a magnetometer sensor 107.

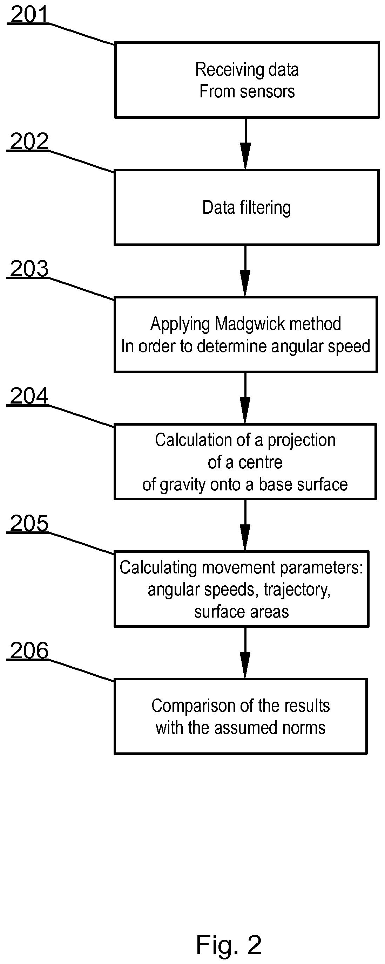

Fig. 2 presents a diagram of the method according to the present invention. The method starts at step 201 from receiving data from sensors 103, 107 at the wearable device.

Typically, data from inertia sensors or magnetic field sensors are subject to noise. Therefore, it is necessary to apply data filtering of the input at step 202 at the wearable device. According to the present invention, there has been applied a low-pass filter operating in a continuous manner, which preferably is a Butterworth, 2nd-order, digital filter having a cutoff frequency of 10 Hz.

Next, the subsequent data processing steps are performed at the external device based on the filtered data received from the wearable device.

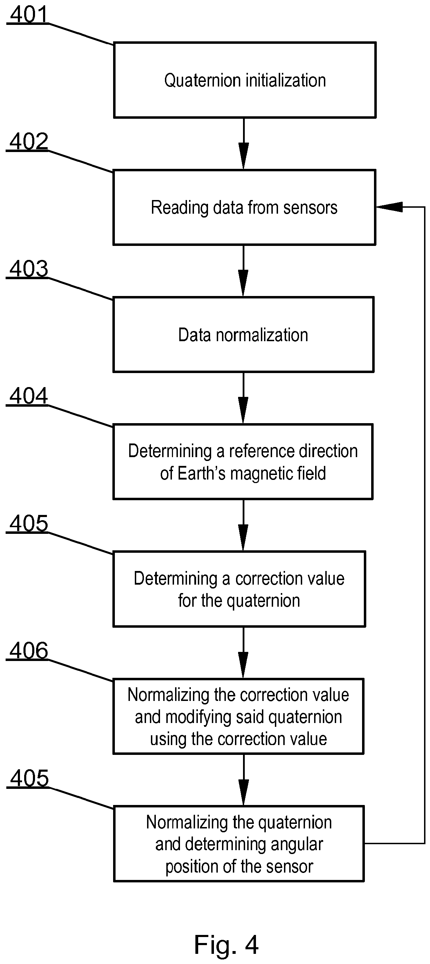

In order to determine angular speed, a particular implementation of a modified Sebastian Madgwick's method has been applied 203, which uses quaternions for representing orientation in three-dimensional space. In mathematics, the quaternions are a number system that extends the complex numbers. They were first described by Irish mathematician William Rowan Hamilton and applied to mechanics in three-dimensional space. The method will be elaborated on in details with reference to Fig. 4.

Sebastian Madgwick's method is based on data from an IMU limited to acceleration and gyroscope data while not taking into account magnetic sensor's data. The method may be divided into the following steps:

- normalization of data from the acceleration sensor;
- gradient descent method application - a corrective step;
- determining a speed of change of the quaternions;
- integration in order to determine quaternions;
- normalization of the quaternions;
- determining an angular position based on the quaternions.

$$\psi = atan2\left(2q_2q_3 - 2q_1q_4, 2q_1^2 + 2q_2^2 - 1\right)$$

$$\theta = -\sin^{-1}\left(2q_2q_4 + 2q_1q_3\right)$$

$$\varphi = atan2\left(2q_3q_4 - 2q_1q_2, 2q_1^2 + 2q_4^2 - 1\right)$$

Based on the calculated quaternions, according to the equations above, there are calculated angles of *roll, pitch, yaw*. They are used in order to determine temporal angular speeds of a rotating rigid body. These are a theoretical creation similar to Euler's angles. They are created by sequential rotations with respect to a base system and not a local system as in case of the Euler's angles. The rotation is effected sequentially against the X axis then Y axis and lastly the Z axis. The order of axes may be different, nevertheless once set it must be followed in subsequent calculations.

At step 204, there is calculated projection of a center of gravity onto a base surface, on which a person is positioned. The equations applied are x=h sin($\alpha$) and y=h sin($\beta$).

At step 205, there are calculated movement parameters such as angular speeds, trajectory, surface areas in the following manner:

- current angular speed in the XY plane

$$\frac{hypot\big(pitch(i) - pitch(i-1), roll(i) - roll(i-1)\big)}{T}$$

where hypot(x,y) is $\sqrt{x^2 + y^2}$

- maximum angular speed in the XY plane
- average angular speed in the XY plane

- trajectory length $\sum_{i=1}^{N} hypot\big(x(i) - x(i-1), y(i) - y(i-1)\big)$ , wherein N is a samples count

- plane area $\sum_{i=1}^{N} \big|\big((x(i-1) - x_0)(y(i) - y_0) - (x(i) - x_0)(y(i-1) - y_0)\big)\big|$ , wherein $x_0$ and $y_0$ is a geometric center of said trajectory.

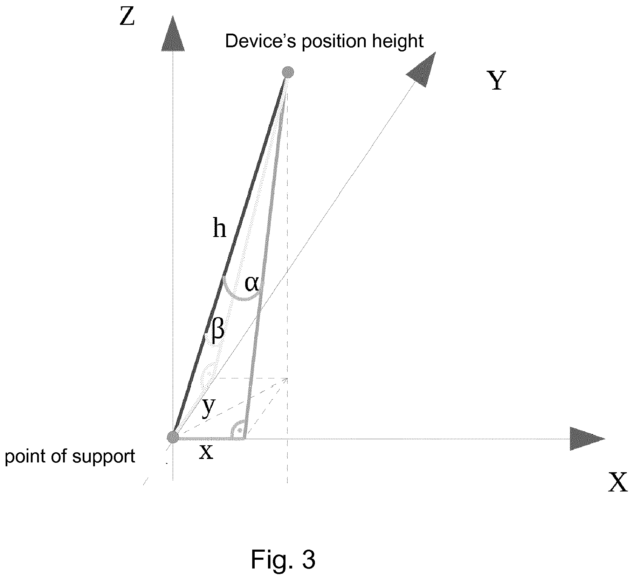

Fig. 3 presents a simplified diagram of a principle of determining coordinates of a projection of a center of gravity onto a base plane/surface.
Ac coding to this diagram, the coordinates are calculated based on the following relations:

$$x = h \sin \alpha$$

$$y = h \sin \beta$$

wherein h denotes a height at which the device is mounted, while the angles $\alpha$ and $\beta$ are the previously defined roll and pitch angles.
The results of the measurements are lastly compared with the assumed norms 206. This allows to determine a state of the person under test.
The norms have been determined based on test evaluations, which were also compared with results of a standard static posturography system. The following table presents such norms:

| | Eyes open firm surface | Eyes closed firm surface | Eyes open soft surface | Eyes closed soft surface |
|---|---|---|---|---|
| Max angular speed [deg/s] | 8,82 | 9,07 | 9,93 | 10,41 |
| Avg angular speed [deg/s] | 0,59 | 0,68 | 0,99 | 1,67 |
| COG plane area [cm$^2$] | 2,84 | 2,37 | 14,13 | 68,15 |
| Trajectory length [cm] | 13,57 | 14,40 | 30,21 | 37,31 |

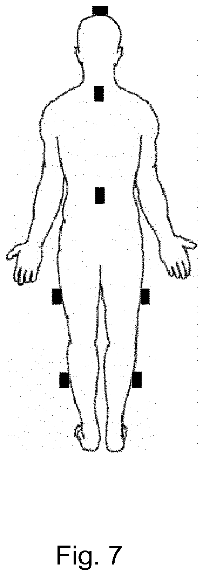

The four tests described above above are performed wherein a person runs two tests with eyes open and two test with eyes closes. The first test is performed on a firm surface while the other is performed on a soft surface such as a foam that makes it more difficult to keep a balance.
Owing to the use of gyroscopes and magetometers, the device has additional capabilities of registering its positioning. This in turn allows for further analysis (going beyond dynamic posturography) such as: head movements tracking, tracking of legs movements or rotations of the person under test, when the device is attached to a particular position at the person's body, as shown in Fig. 7.
Fig. 4 presents a method for determining an angular speed. At step 401 a quaternion is initialized ($q_0$=1, $q_1$=$q_2$=$q_3$=0).

Subsequently, at step 402, there are read data from the three-axis sensors: accelerometer (a), gyroscope (g) and magnetometer (m). Next 403 the accelerometer data are normalized as well as magnetometer data are normalized.

After that, the process moves to step 404, where there is determined a reference direction of Earth's magnetic field by the following calculations:

- $h_x = m_x q_1 q_1 - 2q_1 m_y q_4 + 2q_1 m_z q_3 + m_x q_2 q_2 + 2q_2 m_y q_3 + 2q_2 m_z q_4 - m_x q_3 q_3 - m_x q_4 q_4;$
- $h_y = 2q_1 m_x q_4 + m_y q_1 q_1 - 2q_1 m_z q_2 + 2q_2 m_x q_3 - m_y q_2 q_2 + m_y q_3 q_3 + 2q_3 m z q_4 - m_y q_4 q_4;$
- $b_x = hypot(h_x, h_y)/2$
- $b_z = 0.5(-2q_1 m_x q_3 + 2q_1 m_y q_2 + m_z q_1 q_1 + 2q_2 m_x q_4 - m_z q_2 q_2 + 2q_3 m_y q_4 - m_z q_3 q_3 + m_z q_4 q_4);$

Subsequently, at step 405 there is determined a correction value for a quaternion based on the current value of the quaternion as well as readings from the sensors. The following equations are used for this purpose:

- $s_1 = - 2q_3 (2q_2 q_4 - 2q_1 q_3 - a_x) + 2q_2(2q_1 q_2 + 2q_3 q_4 - a_y) - 2b_z q_3(2b_x (0.5 - q_3 q_3 - q_4 q_4) + 2b_z(q_2 q_4 - q_1 q_3) - m_x) + (-2b_x q_4 + 2b_z q_2)(2b_x(q_2 q_3 - q_1 q_4) + 2b_z(q_1 q_2 + q_3 q_4) - m_y) + 2b_x q_3(2b_x (q_1 q_3 + q_2 q_4) + 2b_z(0.5 - q_2 q_2 - q_3 q_3) - m_z)$
- $s_2 = 2q_4(2q_2 q_4 - 2q_1 q_3 - a_x) + 2q_1(2q_1 q_2 + 2q_3 q_4 - a_y) - 4q_2(1 - 2q_2 q_2 - 2q_3 q_3 - a_z) + 2b_z q_4(2b_x(0.5 - q_3 q_3 - q_4 q_4) + 2b_z(q_2 q_4 - q_1 q_3) - m_x) + (2b_x q_3 + 2b_z q_1)(2b_x(q_2 q_3 - q_1 q_4) + 2b_z(q_1 q_2 + q_3 q_4) - m_y) + (2b_x q_4 - 4b_z q_2)(2b_x(q_1 q_3 + q_2 q_4) + 2b_z(0.5 - q_2 q_2 - q_3 q_3) - m_z)$
- $s_3 = -2q_1(2q_2 q_4 - 2q_1 q_3 - a_x) + 2q_4(2q_1 q_2 + 2q_3 q_4 - a_y) - 4q_3(1 - 2q_2 q_2 - 2q_3 q_3 - a_z) + (-4b_x q_3 - 2b_z q_1)(2b_x(0.5 - q_3 q_3 - q_4 q_4) + 2b_z(q_2 q_4 - q_1 q_3) - m_x) + (2b_x q_2 + 2b_z q_4)(2b_x(q_2 q_3 - q_1 q_4) + 2b_z(q_1 q_2 + q_3 q_4) - m_y) + (2b_x q_1 - 4b_z q_3)(2b_x(q_1 q_3 + q_2 q_4) + 2b_z(0.5 - q_2 q_2 - q_3 q_3) - m_z)$
- $s_4 = 2q_2(2q_2 q_4 - 2q_1 q_3 - a_x) + 2q_3(2q_1 q_2 + 2q_3 q_4 - a_y) + (-4bxq4 + 2b_z q_2) (2b_x(0.5 - q_3 q_3 - q_4 q_4) + 2b_z(q_2 q_4 - q_1 q_3) - m_x) + (-2b_x q_1 + 2b_z q_3)(2b_x(q_2 q_3 - q_1 q_4) + 2b_z(q_1 q_2 + q_3 q_4) - m_y) + 2b_x q_2(2b_x(q_1 q_3 + q_2 q_4) + 2b_z(0.5 - q_2 q_2 - q_3 q_3) - m_z)$

Subsequently, the result of the step 405 is normalized and said quaternion is modified 406 such that:

- $\delta q_1 = 0.5(-q_2 g_x - q_3 g_y - q_4 g_z) - s_1 f_s \beta;$
- $\delta q_2 = 0.5(q1 g_x + q_3 g_z - q_4 g_y) - s_2 f_s \beta;$
- $\delta q_3 = 0.5(q_1 g_y - q_2 g_z + q_4 g_x) - s_3 f_s \beta;$
- $\delta q_4 = 0.5(q_1 g_z + q_2 g_y - q_3 g_x) - s_4 f_s \beta;$
- wherein $f_s$ - is a sampling frequency, $\beta$ - is an attenuation coefficient (0-1)

Next 407, the quaternion is normalized and used in order to determine angular position of the sensor (in general the angular position is calculated, which depending on needs may be converted to angular speed. In case of calculating distance/length or trajectory plane, angular position may suffice).

Said normalization of the quaternion is preferably based on division of each components by a root of the sum of squares of all components e.g.:

- $q_1 = q_1 / sqrt(q_1 q_1 + q_2 q_2 + q_3 q_3 + q_4 q_4)$

Lastly, the steps 402 - 407 are repeated for next data samples from said sensors in a loop.

Figs. 5A-B depict elements related to data transmission scheme according to the present invention.

In particular, Fig. 5A presents a communication interface between said wearable device 502 for dynamic posturography and an external device 501 such as a personal computer, a tablet or a smartphone. The external device comprises means (software, hardware or a combination of both) configured to execute data processing.

The wearable device 502, communicates with the external device 501 preferably in a wireless manner using a suitable communication link such as Wi-Fi or Bluetooth.

The external device 501 connects, to each wearable device for dynamic posturography 502, only twice during each stage of data acquisition (responses testing stage). The first connection is made at the beginning of each test, which may be done by a common start signal preferably also allowing synchronization of said wearable devices 502.

The start command may comprise information on how much time a planned test phase may take (i.e. transmission of expected number of samples). After reception of such start command, the wearable device disconnects from the external device 501 and each sensor independently stores its readings in memory of the wearable device 502. After the test phase, a new connection is made with the external device 501 and the stored data are retrieved by the external device 501. Said retrieval may take a form of a single transmission package spit into frames.

A dedicated frame format has been developed for the purposes of the present invention, as shown in Fig. 5B 503. Such frame 503 comprises the following information:

- id - an identification number of a wearable device,
- nr - sample number,
- accX - acceleration measured in the X axis by an acceleration sensor,
- accY - acceleration measured in the Y axis by an acceleration sensor,
- accZ - acceleration measured in the Z axis by an acceleration sensor,
- gyroX - angular speed measured in the X axis by a gyroscope,
- gyroY - angular speed measured in the Y axis by a gyroscope,
- gyroZ - angular speed measured in the Z axis by a gyroscope,
- magnX - magnetic field measured in the X axis by a magnetometer,
- magnY - magnetic field measured in the Y axis by a magnetometer,
- magnZ - magnetic field measured in the Z axis by a magnetometer.

Optionally, the frame 503 may comprise further data, such as battery charge level.

Preferably, the wearable device 502 transmits data in a 32 bit, Base64 format. All transmitted data values are preferably two byte in a Little endian format. As may be seen in Fig. 5B, there may be present unused bytes/fields that may be utilized by future enhancements of the wearable device 502 and/or the external device 501.

The data transmission scheme is shown in Fig. 6. First, the collected measurement samples are pre-processed in the wearable device 502. This allows to reduce the number of data to be transmitted and thereby reduces power consumption necessary to transmit data, as well as allows high sampling freqency (due to reduction of data to be transmitted for each sample). Due to the fact that some least significant bits of the sensor readouts may contain noise, oversampling and filtering techniques may be used to cancel that noise. Consequently, pre-processed denoised data related to a plurality of samples are transmitted in a single frame 503 wirelessly to the external device 501. Next, the external device, using dedicated software, processes the received data to perform the more power-consuming computing tasks to estimate the angular position, lateral position (including coercion to mechanical model of e.g. a human body) and finally to reconstruct the trajectory of the device or a particular model point.

The aforementioned system and method for dynamic posturography is applied in assessment to quantify the central nervous system adaptive mechanisms involved in the control of posture and balance. Therefore, the invention provides a useful, concrete and tangible result.

The present invention provides a wearable device and a corresponding data processing on input registered by respective sensors. Thus, the machine or transformation test is fulfilled and that the idea is not abstract.

At least parts of the methods according to the invention may be computer implemented. Accordingly, the present invention may take the form of an entirely hardware embodiment, an entirely software embodiment (including firmware, resident software, micro-code, etc.) or an embodiment combining software and hardware aspects that may all generally be referred to herein as a "circuit", "module" or "system".

Furthermore, the present invention may take the form of a computer program product embodied in any tangible medium of expression having computer usable program code embodied in the medium.

It can be easily recognized, by one skilled in the art, that the aforementioned method for dynamic posturography may be performed and/or controlled by one or more computer programs. Such computer programs are typically executed by utilizing the computing resources in a computing device. Applications are stored on a non-transitory medium. An example of a non-transitory medium is a non-volatile memory, for example a flash memory while an example of a volatile memory is RAM. The computer instructions are executed by a processor. These memories are exemplary recording media for storing computer programs comprising computer-executable instructions performing all the steps of the computer-implemented method according the technical concept presented herein.

While the invention presented herein has been depicted, described, and has been defined with reference to particular preferred embodiments, such references and examples of implementation in the foregoing specification do not imply any limitation on the invention. It will, however, be evident that various modifications and changes may be made thereto without departing from the broader scope of the technical concept. The presented preferred embodiments are exemplary only, and are not exhaustive of the scope of the technical concept presented herein.

Accordingly, the scope of protection is not limited to the preferred embodiments described in the specification, but is only limited by the claims that follow.


**Claims**

1.   A system for dynamic posturography of a person, the system comprising:

        - a wearable device (502) comprising:

- a data bus (101) communicatively coupled to a memory (104);
- other components of the wearable device communicatively coupled to the system bus (101) so that they may be managed by a controller (105);
- a communication module (102) to communicate via a wireless communication channel;
- a 3-axis inertial sensor (103) reporting data to the controller (105); and
- a clock module (107) configured to provide timing for sampling data from the 3-axis inertial sensor (103) at a preferred rate;

- and an external device (501) configured to communicate with the wearable device (502) via the wireless communication channel;

said system being **characterised in that**

- said controller (105) of the wearable device (502) is configured to execute the following steps:

- receiving data (201) from a 3-axis inertial sensor (103); and
- filtering of the received data (202);

- said external device (501) is configured to receive from the wearable device (502) the filtered received data and to execute the following steps:

- determining angular speed (203) of the wearable device (502);
- calculating (204) a projection of a center of gravity onto a base surface, on which the person is positioned, wherein x = h sin($\alpha$) and y = h sin($\beta$) wherein h denotes a height at which the device is mounted, while the angles $\alpha$ and $\beta$ are the roll and pitch angles of the angular position of the device calculated based on the data from the 3-axis inertial sensor;
- calculating (205) movement parameters comprising angular speeds and trajectory and surface areas of the wearable device (502); and
- comparing (206) the results of the movement parameters with assumed norms and outputting a result of the comparison as the dynamic posturography parameters.

2. The system according to claim 1 wherein said sampling rate is 20 Hz.

3. The system according to any of previous claims, further comprising a magnetometer (106).

4. The system according to any of previous claims, wherein said filtering (202) is effected by a low-pass filter operating in a continuous manner, which is a Butterworth, 2nd-order, digital filter having a cutoff frequency of 10 Hz.

5. The system according to any of previous claims, wherein said step of calculating (205) movement parameters is effected in the following manner:

- current angular speed in the XY plane

$$\frac{hypot\big(pitch(i)-pitch(i-1),roll(i)-roll(i-1)\big)}{T}$$

where hypot(x,y) is $\sqrt{x^2+y^2}$

- trajectory length $\sum\limits_{i=1}^{N} hypot\big(x(i)-x(i-1),y(i)-y(i-1)\big)$ , wherein N is a samples count

- plane area $\sum\limits_{i=1}^{N} |\big(\big(x(i-1)-x_0\big)\big(y(i)-y_0\big)-\big(x(i)-x_0\big)\big(y(i-1)-y_0\big)\big|$ , wherein $x_0$ and $y_0$ is a geometric center of said trajectory.

6. The system according to any of previous claims, wherein the step of determining angular speed (203) is effected by executing the following steps:

- quaternion initialization (401);
- reading data (402) from sensors (103, 107);
- data normalization (403);
- determining (404) a reference direction of Earth's magnetic field;
- determining a correction value (405) for the quaternion based on their current value and readings from the sensors (103);
- normalizing (406) the correction value (405) and modifying said quaternion using the correction value (405);
- normalizing (407) the quaternion and determining angular position of the sensor.

7. The system according to any of previous claims, wherein said determining a correction value (405) comprises the following equations:

- $s_1 = -2q_3(2q_2q_4 - 2q_1q_3 - a_x) + 2q_2(2q_1q_2 + 2q_3q_4 - a_y) - 2b_zq_3(2b_x(0.5 - q_3q_3 - q_4q_4) + 2b_z(q_2q_4 - q_1q_3) - m_x) + (-2b_xq_4 + 2b_zq_2)(2b_x(q_2q_3 - q_1q_4) + 2b_z(q_1q_2 + q_3q_4) - m_y) + 2b_xq_3(2b_x(q_1q_3 + q_2q_4) + 2b_z(0.5 - q_2q_2 - q_3q_3) - m_z)$
- $s_2 = 2q_4(2q_2q_4 - 2q_1q_3 - a_x) + 2q_1(2q_1q_2 + 2q_3q_4 - a_y) - 4q_2(1 - 2q_2q_2 - 2q_3q_3 - a_z) + 2b_zq_4(2b_x(0.5 - q_3q_3 - q_4q_4) + 2b_z(q_2q_4 - q_1q_3) - m_x) + (2b_xq_3 + 2b_zq_1)(2b_x(q_2q_3 - q_1q_4) + 2b_z(q_1q_2 + q_3q_4) - m_y) + (2b_xq_4 - 4b_zq_2)(2b_x(q_1q_3 + q_2q_4) + 2b_z(0.5 - q_2q_2 - q_3q_3) - m_z)$
- $s_3 = -2q_1(2q_2q_4 - 2q_1q_3 - a_x) + 2q_4(2q_1q_2 + 2q_3q_4 - a_y) - 4q_3(1 - 2q_2q_2 - 2q_3q_3 - a_z) + (-4b_xq_3 - 2b_zq_1)(2b_x(0.5 - q_3q_3 - q_4q_4) + 2b_z(q_2q_4 - q_1q_3) - m_x) + (2b_xq_2 + 2b_zq_4)(2b_x(q_2q_3 - q_1q_4) + 2b_z(q_1q_2 + q_3q_4) - m_y) + (2b_xq_1 - 4b_zq_3)(2b_x(q_1q_3 + q_2q_4) + 2b_z(0.5 - q_2q_2 - q_3q_3) - m_z)$
- $s_4 = 2q_2(2q_2q_4 - 2q_1q_3 - a_x) + 2q_3(2q_1q_2 + 2q_3q_4 - a_y) + (-4bxq4 + 2b_zq_2)(2b_x(0.5 - q_3q_3 - q_4q_4) + 2b_z(q_2q_4 - q_1q_3) - m_x) + (-2b_xq_1 + 2b_zq_3)(2b_x(q_2q_3 - q_1q_4) + 2b_z(q_1q_2 + q_3q_4) - m_y) + 2b_xq_2(2b_x(q_1q_3 + q_2q_4) + 2b_z(0.5 - q_2q_2 - q_3q_3) - m_z)$

8. The system according to any of previous claims, wherein said normalizing (405) the correction value (405) and modifying said quaternion comprises the following equations:

- $\delta q_1 = 0.5(-q_2g_x - q_3g_y - q_4g_z) - s_1f_s\beta$;
- $\delta q_2 = 0.5(q1g_x + q_3g_z - q_4g_y) - s_2f_s\beta$;
- $\delta q_3 = 0.5(q_1g_y - q_2g_z + q_4g_x) - s_3f_s\beta$;
- $\delta q_4 = 0.5(q_1g_z + q_2g_y - q_3g_x) - s_4f_s\beta$;
- wherein $f_s$ - is a sampling frequency, $\beta$ - is an attenuation coefficient (0-1).

9. A method for dynamic posturography of a person by means of a wearable device (502) mounted to a person's body, the method comprising the steps of:

- attaching the wearable device (502) to the person's body;
- at the wearable device (502):

    - receiving data (201) from a 3-axis inertial sensor (103) mounted in the device;
    - filtering of the received data (202);

- at an external device (501) configured to communicate with the wearable device (502) via the wireless communication channel:

    - receiving the filtered data from the wearable device (502);
    - determining angular speed (203) of the wearable device (502);
    - calculating (204) coordinates (x, y) of a projection of a center of gravity onto a base surface, on which the person is positioned, wherein $x = h\sin(\alpha)$ and $y = h\sin(\beta)$ wherein h denotes a height at which the device is mounted, while the angles $\alpha$ and $\beta$ are the roll and pitch angles of the angular position of the device calculated based on the data from the 3-axis inertial sensor;
    - calculating (205) movement parameters comprising angular speeds and trajectory and surface areas of the wearable device (502); and
    - comparing (206) the results of the movement parameters with assumed norms and outputting a result of the comparison as the dynamic posturography parameters.

Fig. 1

201

Receiving data
From sensors

202

Data filtering

203

Applying Madgwick method
In order to determine angular speed

204

Calculation of a projection
of a centre
of gravity onto a base surface

205

Calculating movement parameters:
angular speeds, trajectory,
surface areas

206

Comparison of the results
with the assumed norms

Fig. 2

Fig. 3

401

Quaternion initialization

402

Reading data from sensors

403

Data normalization

404

Determining a reference direction of Earth's magnetic field

405

Determining a correction value for the quaternion

406

Normalizing the correction value and modifying said quaternion using the correction value

405

Normalizing the quaternion and determining angular position of the sensor

Fig. 4

501

External device
(PC, server)

Start

Data request

Samples data

502

Wearable device
(sensors, Fig. 1)

## Fig. 5A

503

| Base64 | | | | Base64 | | | | Base64 | | | | Base64 | | | |
|---|---|---|---|---|---|---|---|---|---|---|---|---|---|---|---|
| id | nr | Not used | Not used | accX | accY | accZ | Not used | gyroX | gyroY | gyroZ | Not used | magnX | magnY | magnZ | Not used |
| 2 | 2 | 2 | 2 | 2 | 2 | 2 | 2 | 2 | 2 | 2 | 2 | 2 | 2 | 2 | 2 |

## Fig. 5B

EP 3 786 976 A1

200 Hz uniform sampling

in dedicated hardware

sensor sampling

| N-6 | N-5 | N-4 | N-3 | N-2 | N-1 | N | N+1 | N+2 | N+3 | N+4 | N+5 | N+6 |

data pre-processing (e.g. aggregation, down-pass filtering)

wireless transmission

in dedicated software

angular position estimation

lateral position estimation, coercion to mechanical model (e.g. human body model)

reconstructed trajectory of the device or particular model point

Fig. 6

Fig. 7

Europäisches
Patentamt

European
Patent Office

Office européen
des brevets

## EUROPEAN SEARCH REPORT

Application Number

EP 19 46 1575

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 10 258 259 B1 (ZETS GARY [US] ET AL) 16 April 2019 (2019-04-16) * column 9, line 58 - column 15, line 9 * ----- | 1-9 | INV. G16H40/67 G16H50/20 |
| A | WO 2010/150260 A1 (MEDICAL RES INFRASTRUCTURE & HEALTH SERVICES FUND TEL AVIV MEDICAL CT) 29 December 2010 (2010-12-29) * page 17, line 15 - page 19, line 13 * ----- | 1-9 | |

TECHNICAL FIELDS
SEARCHED       (IPC)

G16H

The present search report has been drawn up for all claims

| Place of search | Date of completion of the search | Examiner |
|---|---|---|
| Munich | 7 February 2020 | Rinelli, Pietro |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

............................................................................

& : member of the same patent family, corresponding
document

EPO FORM 1503 03.82 (P04C01)

## ANNEX TO THE EUROPEAN SEARCH REPORT
## ON EUROPEAN PATENT APPLICATION NO.

EP 19 46 1575

This annex lists the patent family members relating to the patent documents cited in the above-mentioned European search report.
The members are as contained in the European Patent Office EDP file on
The European Patent Office is in no way liable for these particulars which are merely given for the purpose of information.

07-02-2020

| Patent document cited in search report | | Publication date | Patent family member(s) | | Publication date |
|---|---|---|---|---|---|
| US 10258259 | B1 | 16-04-2019 | NONE | | |
| WO 2010150260 | A1 | 29-12-2010 | CA | 2765782 A1 | 29-12-2010 |
| | | | EP | 2445405 A1 | 02-05-2012 |
| | | | US | 2012101411 A1 | 26-04-2012 |
| | | | WO | 2010150260 A1 | 29-12-2010 |

EPO FORM P0459

For more details about this annex : see Official Journal of the European Patent Office, No. 12/82

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20140081177 A1